# EUROPEAN PATENT SPECIFICATION

(11) **EP 2 949 354 B1**
(45) Date of publication and mention of the grant of the patent: **21.12.2016**
(21) Application number: 14170396.7
(22) Date of filing: 28.05.2014
(51) Int. Cl.: A61M 5/142

(54) **Fluid dispensing device**
Flüssigkeitsspendevorrichtung
Distributeur de fluides

(43) Date of publication of application: 02.12.2015
(73) Proprietor: Wang, Hong Jen, Kaohsiung 80052 (TW)
(72) Inventor: Wang, Hong Jen, Kaohsiung 80052 (TW)
(74) Representative: Beck & Rössig European Patent Attorneys

(56) References cited:
- WO-A2-03/022328
- US-A1- 2012 012 618
- US-A1- 2013 165 758

## Description

The invention relates to a fluid dispensing device including a pressurizing device for supplying a fluid to a patient without gravity.

Typical fluid dispensing devices comprise a pressurizing device for forcing the fluid into a patient without gravity.

However, the piston may not effectively pump the fluid.

The invention provides fluid dispensing device as defined in the claims.

In the drawings:
FIG. 1 is a perspective view of a fluid dispensing device;
FIG. 2 is a partial exploded view of the fluid dispensing device;
FIG. 3 is another partial perspective view of the device; and
FIGS. 4, 5, 6 are partial cross sectional views of the device.

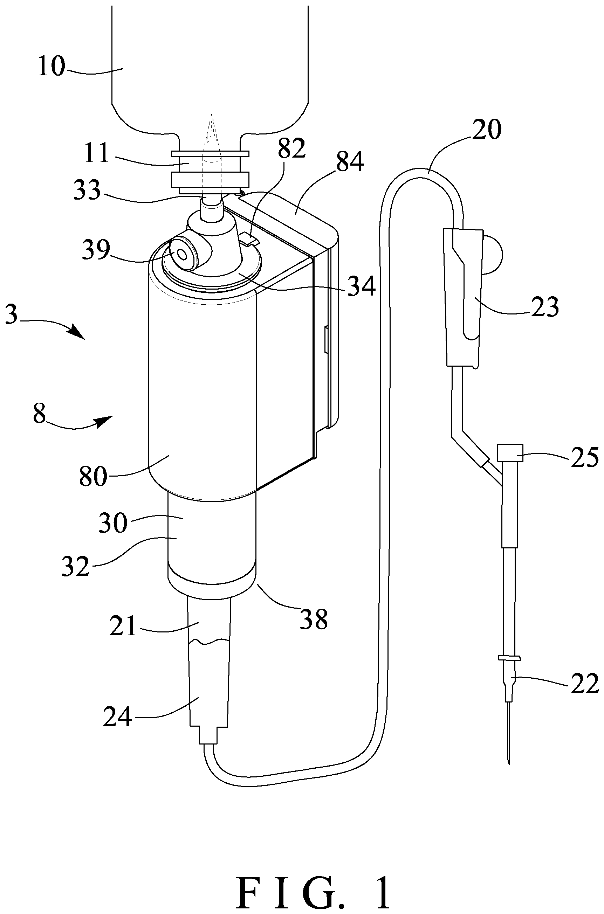

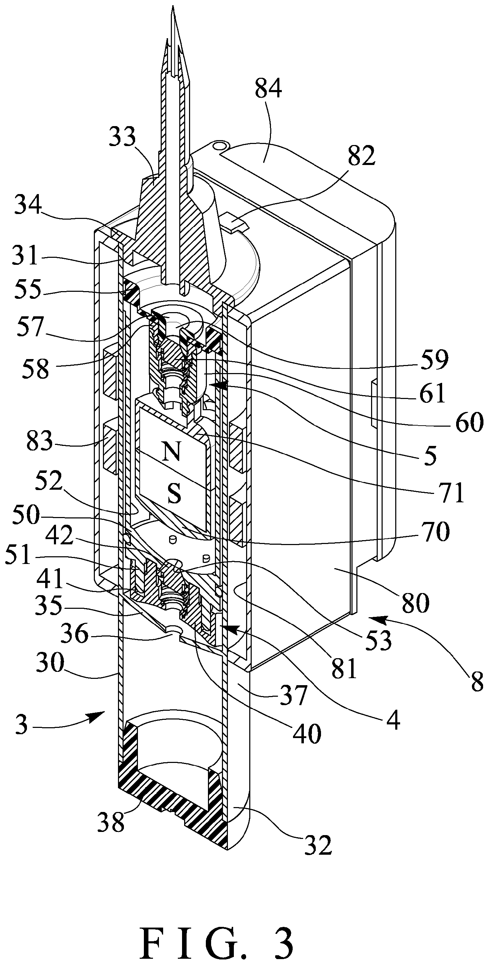

Referring to FIGS. 1-4, a fluid dispensing device comprises a bottle 10 for receiving a medicinal fluid, and a discharge tube 20 having a hypodermic needle 21 for coupling to the bottle 10, and having an injection needle 22 for penetrating into patient's body. A clamp valve 23 is attached onto the tube 20, and a flow indicating device 24 is positioned in the tube 20 for observing the fluid. A relief valve 25 is attached to the tube 20.

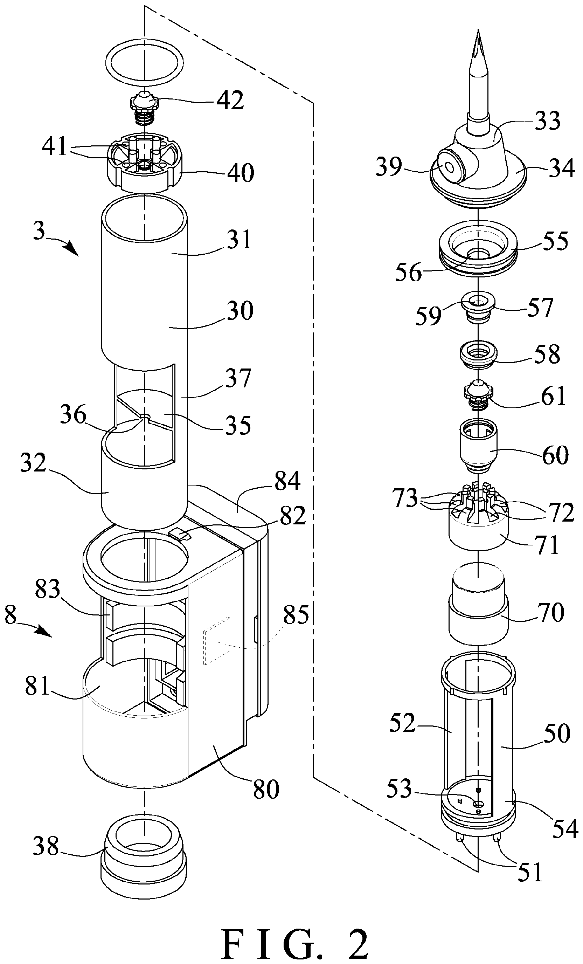

A pressurizing device 3 is coupled between the bottle 10 and the tube 20 for pressurizing the fluid (FIGS. 2-4), and includes a container 30, a hypodermic needle 33 secured to an upper portion 31 of the container 30 for engaging into the lower portion 11 of the bottle 10, the hypodermic needle 33 includes a flap 34 and a relief port 39. The container 30 includes a partition 35 formed in the lower portion 32 or middle portion 37 and having a hole 36, a cover 38 attached to the lower portion 32 of the container 30. The hypodermic needle 21 is engaged through the cover 38 and into the container 30.

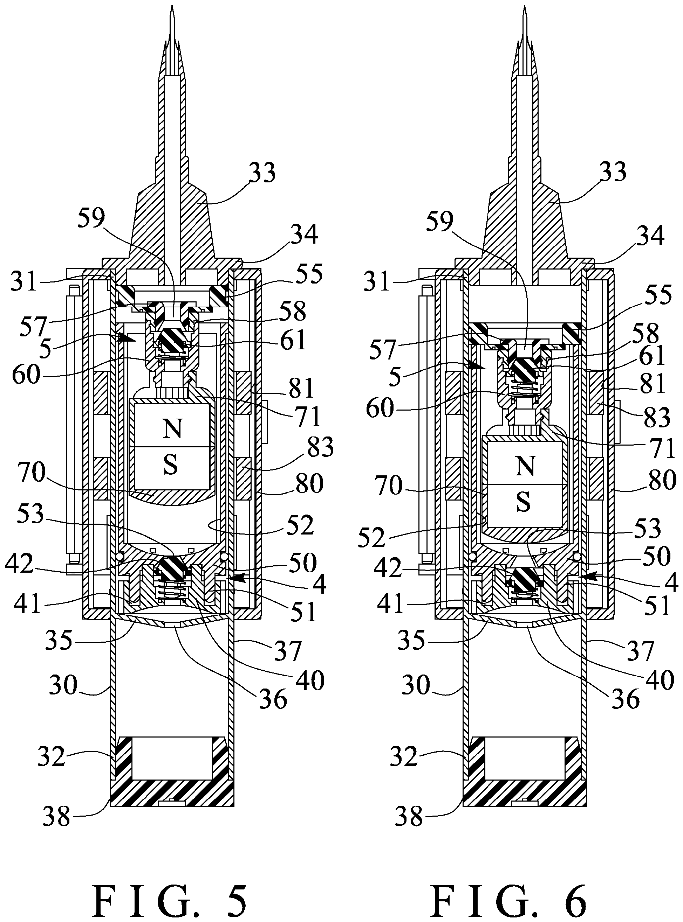

A first or lower check valve 4 includes a base 40 engaged in the container 30 and seated on the partition 35, and a housing 50 is engaged in the container 30 and movable toward or away from the base 40, the housing 50 may include one or more pins 51 for slidably engaging with the cavities 41 of the base 40, and includes a compartment 52 for receiving the fluid, and a valve seat or exit port 53 is formed in the lower portion 54 of the housing. A spring biased plug 42 is engaged onto the base 40 and between the base 40 and the housing 50 for engaging with the exit port 53 of the housing 50. When the housing 50 is moved toward the base 40, the fluid in the housing 50 may disengage the plug 42 from the exit port 53 (FIG. 5) to force the fluid to flow out of the housing 50 and into the hypodermic needle 21. The base 40 and the exit port 53 in the housing 50 and the plug 42 may form the first or lower check valve 4.

A second or upper check valve 5 includes a cap 55 secured on top of the housing 50 and having one or more orifices 56 (FIG. 2), a gasket 57 is engaged into the orifice 56 of the cap 55 and onto the cap 55, and a lock 58 is attached to the gasket 57 and engaged with the lower portion of the cap 55 for securing the gasket 57 and the lock 58 to the cap 55, and the gasket 57 includes a passage 59 for allowing the fluid to flow through the cap 55.

A casing 60 is disposed in the housing 50 and secured to the cap 55 and/or the gasket 57 and/or the lock 58, another spring biased plug or valve piece 61 is engaged in the casing 60 and engaged with the gasket 57 or the cap 55 for blocking the passage 59 of the gasket 57 and for controlling the fluid to flow through the cap 55 and/or the gasket 57.

A core or magnetic member 70 is attached to the cap 55 and/or the casing 60 with a bracket 71 which is attached to the casing 60.

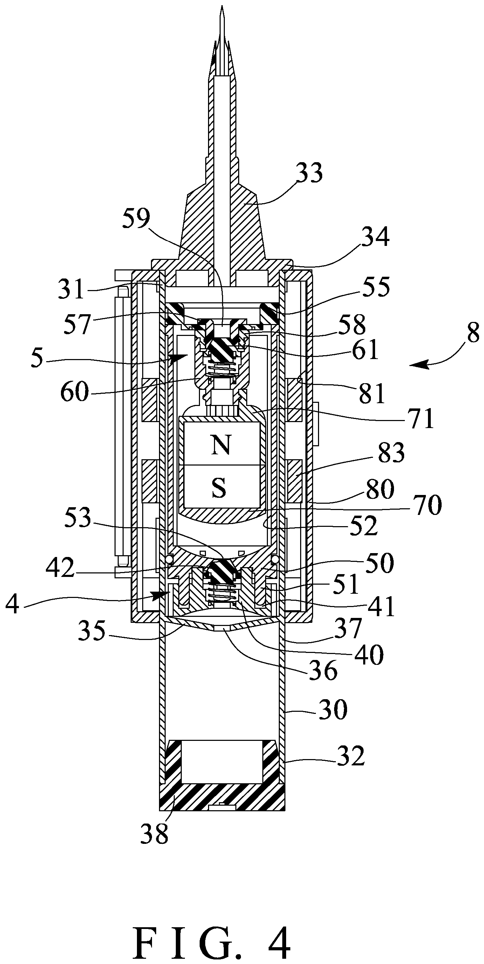

The bracket 71 includes one or more slots 72 (FIG. 2) for forming one or more fingers 73 and for securing the bracket 71 and the core 70 to the casing 60. The fluid may flow through the orifice 56 of the cap 55 (FIG. 4) and/or the passage 59 of the gasket 57 and into and out of the casing 60 through the slots 72 of the bracket 71, and may then flow into the housing 50 and out through the exit port 53 of the housing 50. The core and the bracket 71 and the casing 60 and the cap 55 are secured to the housing 50. An actuating device 8 includes a receptacle 80 having a chamber 81 for receiving the container 30, the receptacle 80 includes one or more catches 82 for engaging with the flap 34 of the needle 33, one or more coils 83 are attached to the receptacle 80 and disposed around the core 70.

One or more batteries 84 and/or a control device 85 are attached to the receptacle 80 for actuating the coil 83. When the core 70 and the housing 50 are moved toward the base 40 and the partition 35 or away from the needle 33 (FIG. 5), the fluid in the housing 50 may force the plug 42 away from the exit port 53 and may flow out of the housing 50 and through the base 40 and the hole 36 of the partition 35 and to into the needle 21. On the contrary, when the core 70 and the housing 50 are moved away from the base 40 and the partition 35 or toward the needle 33, the plug 42 may be engaged with the exit port 53, and the fluid in the upper portion 31 of the container 30 may force the valve piece 61 away from the gasket 57 and may flow into and out of the casing 60 and the slots 72 of the bracket 71, and then into the housing 50. The valve piece 61 may form the second or upper check valve 5.

## Claims

1. Fluid dispensing device comprising:
a bottle (10) for receiving a fluid,
a tube (20), and
a pressurizing device (3) coupled between the bottle (10) and the tube (20),
**characterised in that:**
the pressurizing device (3) includes a container (30) coupled between the bottle and the tube, a base (40) disposed in the container (30), a housing (50) engaged in the container (30) and movable toward and away from the base (40) and having a compartment (52), and an exit port (53) communicating with the compartment (52) of the housing, a plug (42) engaged between the base (40) and the housing (50) for engaging with the exit port (53) of the housing, a cap (50) mounted on the housing (50) and having an orifice, a casing (60) disposed in the housing (50) and attached to the cap (55), a valve piece (61) engaged in the casing (60) for engaging with the orifice of the cap (55), a core (70) attached to the casing (60), and at least one coil (83) attached to the container (30) to move the core (70) and the casing (60) and the cap (55) and the housing (50) in the container (30), and the plug (42) is forced to move away from the exit port (53) of the housing (50) and to control the fluid to flow out of the housing (50) when the housing is moved toward the base (40), and the valve piece (61) is forced away from the orifice of the cap (55) when the housing (50) is moved away from the base (40).

2. Fluid dispensing device as claimed in claim 1, **characterised in that** the cap (55) includes a gasket (57) engaged into the orifice of the cap, and the gasket (57) includes a passage for engaging with the valve piece (61).

3. Fluid dispensing device as claimed in claim 2, **characterised in that** the cap (55) has a lock (58) attached to the gasket (57) and engaged with the cap.

4. Fluid dispensing device as claimed in one of claims 1 to 3, **characterised in that** the core (70) is attached to a bracket (71), and the bracket (71) is secured to the casing (60).

5. Fluid dispensing device as claimed in claim 4, **characterised in that** the bracket (71) includes at least one slot (72) for forming at least one finger.

6. Fluid dispensing device as claimed in one of claims 1 to 5, **characterised in that** the container (30) includes a partition (35) provided in the container for engaging with the base (40), and the partition includes a hole (36).

7. Fluid dispensing device as claimed in one of claims 1 to 6, **characterised in that** the base (40) includes at least one cavity (41), and the housing (50) includes at least one pin (51) for engaging with the cavity (41) of the base.

8. Fluid dispensing device as claimed in one of claims 1 to 7, **characterised in that** the pressurizing device (3) includes a receptacle (80) having a chamber (18) for receiving the container (30), and the coil (83) is attached to the receptacle (80).

9. Fluid dispensing device as claimed in claim 8, **characterised in that** the container (30) includes a hypodermic needle provided (33) on an upper portion (31) of the container and having a flap (34), and the receptacle (80) includes at least one catch (82) engaged with the flap (34).

10. Fluid dispensing device as claimed in claim 8 or 9, **characterised in that** a battery (84) is attached to the receptacle (80).

## Patentansprüche

1. Fluid-Abgabevorrichtung, umfassend:
eine Flasche (10) zur Aufnahme eines Fluids,
ein Rohr (20), und
eine Druckvorrichtung (3), die zwischen der Flasche (10) und dem Rohr (20) angeordnet ist,
**dadurch gekennzeichnet, dass**
die Druckvorrichtung (3) einen Behälter (30) aufweist, der zwischen der Flasche und dem Rohr angeordnet ist, eine Basis (40), die in dem Behälter (30) angeordnet ist, ein Gehäuse (50), das in den Behälter (30) eingesetzt und auf die Basis (40) zu und davon weg bewegbar ist und eine Kammer (52) aufweist, und eine Ausgangsöffnung (53), die mit der Kammer (52) des Gehäuses in Verbindung steht, einen Stopfen (42), der zwischen die Basis (40) und das Gehäuse (50) eingesetzt ist, um mit der Ausgangsöffnung (53) des Gehäuses in Eingriff zu kommen, eine Kappe (50) die an dem Gehäuse (50) montiert ist und eine Öffnung aufweist, eine Kapsel (60), die in dem Gehäuse (50) angeordnet und an der Kappe (55) befestigt ist, ein Ventilstück (61), das in die Kapsel (60) eingesetzt ist, um mit der Öffnung der Kappe (55) in Eingriff zu kommen, einen Kern (70), der an der Kapsel (60) befestigt ist, und mindestens eine Wicklung (83), die an dem Behälter (30) befestigt ist, um den Kern (70) und die Kapsel (60) und die Kappe (55) und das Gehäuse (50) in dem Behälter (30) zu bewegen, und der Stopfen (42) dazu gezwungen wird, sich von der Ausgangsöffnung (53) des Gehäuses (50) weg zu bewegen und den Fluidstrom aus dem Gehäuse (50) zu steuern, wenn das Gehäuse in Richtung der Basis (40) bewegt wird, und das Ventilstück (61) von der Öffnung der Kappe (55) weggedrückt wird, wenn das Gehäuse (50) von der Basis (40) wegbewegt wird.

2. Fluid-Abgabevorrichtung nach Anspruch 1, **dadurch gekennzeichnet, dass** die Kappe (55) eine Dichtung (57) aufweist, die in die Öffnung der Kappe eingesetzt ist, und die Dichtung (57) einen Durchgang aufweist, um mit dem Ventilstück (61) den Eingriff zu kommen.

3. Fluid-Abgabevorrichtung nach Anspruch 2, **dadurch gekennzeichnet, dass** die Kappe (55) eine Verriegelung (58) aufweist, die an der Dichtung (57) befestigt ist und mit der Kappe in Eingriff steht.

4. Fluid-Abgabevorrichtung nach einem der Ansprüche 1 bis 3, **dadurch gekennzeichnet, dass** der Kern (70) an einer Halterung (71) befestigt ist und die Halterung (71) an der Kapsel (60) gesichert ist.

5. Fluid-Abgabevorrichtung nach Anspruch 4, **dadurch gekennzeichnet, dass** die Halterung (71) mindestens einen Schlitz (72) aufweist, um mindestens einen Finger zu bilden.

6. Fluid-Abgabevorrichtung nach einem der Ansprüche 1 bis 5, **dadurch gekennzeichnet, dass** der Behälter (30) eine Trennwand (35) aufweist, die in dem Behälter angeordnet ist, um mit der Basis (40) in Eingriff zu kommen, und die Trennwand ein Loch (36) aufweist.

7. Fluid-Abgabevorrichtung nach einem der Ansprüche 1 bis 6, **dadurch gekennzeichnet, dass** die Basis (40) mindestens einen Hohlraum (41) aufweist, und das Gehäuse (50) mindestens einen Stift (51) zum Eingriff mit dem Hohlraum (41) der Basis aufweist.

8. Fluid-Abgabevorrichtung nach einem der Ansprüche 1 bis 7, **dadurch gekennzeichnet, dass** die Druckvorrichtung (3) eine Aufnahme (80) mit einer Kammer (18) zum Aufnehmen des Behälters (30) aufweist, und die Wicklung (83) an der Aufnahme (80) befestigt ist.

9. Fluid-Abgabevorrichtung nach Anspruch 8, **dadurch gekennzeichnet, dass** der Behälter (30) eine Injektionsnadel aufweist, die an einem oberen Abschnitt (31) des Behälters angeordnet ist und einen Deckel (34) aufweist, und die Aufnahme (80) mindestens eine Arretierung (82) aufweist, die mit dem Deckel (34) in Eingriff steht.

10. Fluid-Abgabevorrichtung nach Anspruch 8 oder 9, **dadurch gekennzeichnet, dass** eine Batterie (84) an der Aufnahme (80) befestigt ist.

## Revendications

1. Dispositif distributeur de fluide comprenant :
un flacon (10) pour recevoir un fluide,
un tube (20) et
un dispositif de pressurisation (3) couplé entre le flacon (10) et le tube (20),
**caractérisé en ce que** le dispositif de pressurisation (3) comprend un conteneur (30) couplé entre le flacon et le tube, une base (40) disposée dans le conteneur (30), un boîtier (50) enclenché dans le conteneur (30) et mobile vers et en s'éloignant de la base (40) et ayant un compartiment (52) et un orifice de sortie (53) qui communique avec le compartiment (52) du boîtier, un bouchon (42) enclenché entre la base (40) et le boîtier (50) pour s'enclencher avec l'orifice de sortie (53) du boîtier, un capuchon (50) monté sur le boîtier (50) et qui a un orifice, un tubage (60) disposé dans le boîtier (50) et attaché au capuchon (55), une pièce de valve (61) enclenchée dans le tubage (60) pour s'enclencher avec l'orifice du capuchon (55), un noyau (70) attaché au tubage (60) et au moins une bobine (83) attachée au conteneur (30) pour déplacer le noyau (70) et le tubage (60) et le capuchon (55) et le boîtier (50) dans le conteneur (30) et le bouchon (42) est forcé de s'éloigner de l'orifice de sortie (53) du boîtier (50) et de contrôler le fluide pour s'écouler hors du boîtier (50) lorsque le boîtier est déplacé vers la base (40) et la pièce de valve (61) est forcée à s'éloigner de l'orifice du capuchon (55) lorsque le boîtier (50) est éloigné de la base (40).

2. Dispositif distributeur de fluide selon la revendication 1, **caractérisé en ce que** le capuchon (55) comprend un joint d'étanchéité (57) enclenché dans l'orifice du capuchon et le joint d'étanchéité (57) comprend un passage pour s'enclencher avec la pièce de valve (61).

3. Dispositif distributeur de fluide selon la revendication 2, **caractérisé en ce que** le capuchon (55) a un pêne (58) attaché au joint d'étanchéité (57) et enclenché avec le capuchon.

4. Dispositif distributeur de fluide selon l'une des revendications 1 à 3, **caractérisé en ce que** le noyau (70) est attaché à un collier (71) et le collier (71) est fixé au tubage (60).

5. Dispositif distributeur de fluide selon la revendication 4, **caractérisé en ce que** le collier (71) comprend au moins une fente (72) pour former au moins un doigt.

6. Dispositif distributeur de fluide selon l'une des revendications 1 à 5, **caractérisé en ce que** le conteneur (30) comprend une séparation (35) prévue dans le conteneur pour s'enclencher avec la base (40) et la séparation comprend un trou (36).

7. Dispositif distributeur de fluide selon l'une des revendications 1 à 6, **caractérisé en ce que** la base (40) comprend au moins une cavité (41) et le boîtier (50) comprend au moins une broche (51) pour s'enclencher avec la cavité (41) de la base.

8. Dispositif distributeur de fluide selon l'une des revendications 1 à 7, **caractérisé en ce que** le dispositif de pressurisation (3) comprend un réceptacle (80) qui a une chambre (18) pour recevoir le conteneur (30) et la bobine (83) est attachée au réceptacle (80).

9. Dispositif distributeur de fluide selon la revendication 8, **caractérisé en ce que** le conteneur (30) comprend une aiguille hypodermique prévue (33) sur une portion supérieure (31) du conteneur et qui a un volet (34) et le réceptacle (80) comprend au moins un ergot (82) enclenché avec le volet (34).

10. Dispositif distributeur de fluide selon la revendication 8 ou 9, **caractérisé en ce qu'**une pile (84) est attachée au réceptacle (80).
